# EUROPEAN PATENT APPLICATION

(11) **EP 2 518 165 A1**
(43) Date of publication of application: **31.10.2012**
(21) Application number: 12166072.4
(22) Date of filing: 27.04.2012
(51) Int. Cl.: C12Q 1/68

(54) **Method for simultaneously detecting gene mutations of acetaldehyde dehydrogenase 2 and alcohol dehydrogenase 2**

(30) Priority: 28.04.2011 JP 2011102333
(71) Applicant: ARKRAY, Inc., Kyoto-shi, Kyoto 601-8045 (JP)
(72) Inventor: Hirai, Mitsuharu, Kyoto-shi, Kyoto 602-0008 (JP); Iguchi, Aki, Kyoto-shi, Kyoto 602-0008 (JP)
(74) Representative: Jones, Elizabeth Louise

(57) **Abstract**

A probe for detection of at least 1 type of genetic polymorphism of the ALDH2 gene rs671 and the ADH2 gene rs1229984, comprising at least 1 type of fluorescently labeled oligonucleotide selected from (P1) to (P3') below:
(P1) an oligonucleotide comprising a nucleotide sequence complementary to a nucleotide sequence of 11 to 50 consecutive nucleotides containing nucleotides 241 to 251 in SEQ ID NO: 1 or 2 or a homologous sequence thereof, wherein the nucleotide corresponding to the nucleotide at position 241 is a cytosine labeled with a fluorescent dye;
(P1') an oligonucleotide comprising a nucleotide sequence which hybridizes with a nucleotide sequence of 11 to 50 consecutive nucleotides containing nucleotides 241 to 251 in SEQ ID NO:1 or 2 under stringent conditions, wherein the nucleotide corresponding to the nucleotide at position 241 is a cytosine labeled with a fluorescent dye;
(P2) an oligonucleotide comprising a nucleotide sequence of 11 to 50 consecutive nucleotides containing nucleotides 251 to 261 in SEQ ID NO:1 or 2 or a homologous sequence thereof, wherein the nucleotide corresponding to the nucleotide at position 261 is a cytosine labeled with a fluorescent dye;
(P2') an oligonucleotide comprising a nucleotide sequence of 11 to 50 consecutive nucleotides containing nucleotides 251 to 261 in SEQ ID NO:1 or 2 or a nucleotide sequence which hybridizes with the complementary strand of SEQ ID NO: 1 or 2 under stringent conditions, wherein the nucleotide corresponding to the nucleotide at position 261 is a cytosine labeled with a fluorescent dye;
(P3) an oligonucleotide comprising a nucleotide sequence of 12 to 50 consecutive nucleotides containing nucleotides 201 to 212 in SEQ ID NO: 13 or a homologous sequence thereof, wherein the nucleotide corresponding to the nucleotide at position 212 is a cytosine labeled with a fluorescent dye; and
(P3') an oligonucleotide comprising a nucleotide sequence of 12 to 50 consecutive nucleotides containing nucleotides 201 to 212 in SEQ ID NO: 13 or a nucleotide sequence which hybridizes with the complementary strand of SEQ ID NO: 13 under stringent conditions, wherein the nucleotide corresponding to the nucleotide at position 212 is a cytosine labeled with a fluorescent dye.

## Description

### TECHNICAL FIELD

The present invention relates to a method for detecting gene mutations of acetaldehyde dehydrogenase 2 (ALDH2) and alcohol dehydrogenase 2 (ADH2), and a nucleic acid probe and a kit therefor.

### BACKGROUND ART

Representative examples of enzymes involved in alcohol metabolism include ADH (alcohol dehydrogenase) and ALDH (aldehyde dehydrogenase). ADH metabolizes ethanol to acetaldehyde, and ALDH metabolizes acetaldehyde to acetic acid.

There are 3 types of ADH, that is, ADH1, ADH2 and ADH3, and, as genetic polymorphisms involved in phenotypes of the ADH2 gene, there are ADH2* 1 (WT), ADH2*2 and ADH2*3 (F Tanaka et al., Hepatology, Volume 23, Issue 2, pages 234-239, February 1996). In the Japanese population, their frequencies are *1/*1: 8.4%, *1/*2: 34.9% and *2/*2: 56.7%, and *3 hardly exists (Kyoko Saito et al., "Do ethanol metabolic enzymes modify the relationship between alcohol drinking and the blood pressure?" 15th Meeting of Society of Blood Pressure Control, Subject 3 (http://www.healthcare.omron.co.jp/medical/study/pdf/past15_03.pdf)).
As genetic polymorphisms involved in phenotypes of the ALDH2 gene, there are *1(WT) and *2 (F Tanaka et al., Hepatology, Volume 23, Issue 2, pages 234-239, February 1996), and their frequencies in Japanese are *1/*1: 52.8%, *1/*2: 40.9% and *2/*2: 6.3% (Kyoko Saito et al., "Do ethanol metabolic enzymes modify the relationship between alcohol drinking and the blood pressure?" 15th Meeting of Society of Blood Pressure Control, Subject 3).

Amongst these genetic polymorphisms, ADH2*2 and ALDH2 are reported to be involved in alcohol dependence, liver diseases and liver cancer (F Tanaka et al., Hepatology, Volume 23, Issue 2, pages 234-239, February 1996; Keitaro Matsuo et al., Research Report of The Uehara Memorial Foundation, 23 (2009), Molecular Epidemiologic Research for Evaluation of Influences of the Genetic Background and the Drinking Habit on the Risk of Developing Pancreatic Cancer (http://ueharazaidan.yoshida-p.net/houkokushu/Vol.23/pdf/046_report.pdt)).
CYP2E1 is also reported to have the same function as ADH2 and ALDH2 (JP 2008-79604 A).

JP 2008-79604 A describes a primer set and a probe set for detection of genetic mutations (in the ALDH2, ADH2 and CYP2E1 genes) involved in the capacity to metabolize alcohol and alcohol tolerance, by hybridization. However, there are problems in that (1) the detection needs to be carried out using one reaction system (one tube) per mutation involved in alcohol metabolism or the like, which is laborious, costly and time-consuming; (2) since DNA purified from saliva/whole blood needs to be used, much labour, cost and time are required, such that the measurement cannot be carried out simply; and (3) since an amplification product needs to be handled for microarray analysis, there is the risk of contamination of the amplification product by another sample.

On the other hand, there are known methods wherein a region containing a mutation is amplified by PCR and a fluorescently labeled nucleic acid probe is used to carry out melting curve analysis, followed by analyzing the mutation based on the result of the melting curve analysis (JP 2001-286300 A and JP 2002-119291 A). However, these documents only teach that the probe is designed such that, when a quenching probe labeled at its end with a fluorescent dye is hybridized with a target nucleic acid, a plurality of base pairs in the probe-nucleic acid hybrid form at least one GC pair at the end of the hybrid. Further, these methods have a problem in that the methods are not necessarily applicable to arbitrary sequences.

### SUMMARY OF THE INVENTION

The present invention aims to provide probes effective for detecting rs671, which is a genetic polymorphism of acetaldehyde dehydrogenase 2 (ALDH2), and rs1229984, which is a genetic polymorphism of alcohol dehydrogenase 2 (ADH2), and to provide a method for detecting these genetic polymorphisms at the same time (i.e. simultaneously) and a kit therefor.

The present inventors discovered that, by designing probes based on specific regions containing the polymorphism of the acetaldehyde dehydrogenase 2 (ALDH2) gene rs671 and the polymorphism of the alcohol dehydrogenase 2 (ADH2) gene rs1229984 and carrying out melting curve analysis using the probes, the mutations can be detected, thereby completing the present invention.

That is, the present invention is as follows.
(1) A probe for detection of at least 1 type of genetic polymorphism of the ALDH2 gene rs671 and the ADH2 gene rs1229984, comprising at least 1 type of fluorescently labeled oligonucleotide selected from (P1) to (P3') below:
   (P1) an oligonucleotide comprising a nucleotide sequence complementary to a nucleotide sequence of 11 to 50 consecutive nucleotides containing nucleotides 241 to 251 in SEQ ID NO:1 or 2 or a homologous sequence thereof, wherein the nucleotide corresponding to the nucleotide at position 241 is a cytosine labeled with a fluorescent dye;
   (P1') an oligonucleotide comprising a nucleotide sequence which hybridizes with a nucleotide sequence of 11 to 50 consecutive nucleotides containing nucleotides 241 to 251 in SEQ ID NO:1 or 2 under stringent conditions, wherein the nucleotide corresponding to the nucleotide at position 241 is a cytosine labeled with a fluorescent dye;
   (P2) an oligonucleotide comprising a nucleotide sequence of 11 to 50 consecutive nucleotides containing nucleotides 251 to 261 in SEQ ID NO:1 or 2 or a homologous sequence thereof, wherein the nucleotide corresponding to the nucleotide at position 261 is a cytosine labeled with a fluorescent dye;
   (P2') an oligonucleotide comprising a nucleotide sequence of 11 to 50 consecutive nucleotides containing nucleotides 251 to 261 in SEQ ID NO:1 or 2 or a nucleotide sequence which hybridizes with the complementary strand of SEQ ID NO: 1 or 2 under stringent conditions, wherein the nucleotide corresponding to the nucleotide at position 261 is a cytosine labeled with a fluorescent dye;
   (P3) an oligonucleotide comprising a nucleotide sequence of 12 to 50 consecutive nucleotides containing nucleotides 201 to 212 in SEQ ID NO:13 or a homologous sequence thereof, wherein the nucleotide corresponding to the nucleotide at position 212 is a cytosine labeled with a fluorescent dye; and
   (P3') an oligonucleotide comprising a nucleotide sequence of 12 to 50 consecutive nucleotides containing nucleotides 201 to 212 in SEQ ID NO:13 or a nucleotide sequence which hybridizes with the complementary strand of SEQ ID NO:13 under stringent conditions, wherein the nucleotide corresponding to the nucleotide at position 212 is a cytosine labeled with a fluorescent dye.
(2) The probe according to (1), wherein
   said oligonucleotides (P1) and (P1') have the nucleotide corresponding to the nucleotide at position 241 labeled with a fluorescent dye at the first, second or third position from the 3' end;
   said oligonucleotides (P2) and (P2') have the nucleotide corresponding to the nucleotide at position 261 labeled with a fluorescent dye at the first, second or third position from the 3' end; and
   said oligonucleotides (P3) and (P3') have the nucleotide corresponding to the nucleotide at position 212 labeled with a fluorescent dye at the first, second or third position from the 3' end.
(3) The probe according to (1), wherein
   said oligonucleotides (P1) and (P1') have the nucleotide corresponding to the nucleotide at position 241 labeled with a fluorescent dye at the 3' end;
   said oligonucleotides (P2) and (P2') have the nucleotide corresponding to the nucleotide at position 261 labeled with a fluorescent dye at the 3' end; and
   said oligonucleotides (P3) and (P3') have the nucleotide corresponding to the nucleotide at position 212 labeled with a fluorescent dye at the 3' end.
(4) The probe for detecting a polymorphism according to any one of (1) to (3), wherein said oligonucleotide emits fluorescence when the oligonucleotide is not hybridized with a target sequence, and the fluorescence intensity decreases or increases when the oligonucleotide is hybridized with the target sequence.
(5) The probe for detecting a polymorphism according to (4), wherein the fluorescence intensity decreases when said oligonucleotide is hybridized with the target sequence.
(6) The probe for detecting a polymorphism according to any one of (1) to (5), wherein said oligonucleotides (P1) to (P3') have 12 to 30 consecutive nucleotides.
(7) The probe for detecting a polymorphism according to any one of (1) to (5), wherein said oligonucleotides (P1) to (P3') have 15 to 30 consecutive nucleotides.
(8) The probe for detecting a polymorphism according to any one of (1) to (5), wherein said oligonucleotides (P1) to (P3') have 18 to 30 consecutive nucleotides.
(9) The probe for detecting a polymorphism according to any one of (1) to (8), wherein said probe is a probe for melting curve analysis.
(10) A method for detecting at least one polymorphism selected from the group consisting of the polymorphism of the ALDH2 gene rs671 and the polymorphism of the ADH2 gene rs1229984, by using at least one probe for detecting a polymorphism according to any one of (1) to (9).
(11) The method for detecting a polymorphism(s) according to (10), comprising
   (I) bringing the probe for detection of a polymorphism(s) according to (1) to (9) into contact with a single-stranded nucleic acid in a sample, to allow hybridization of said fluorescently labeled oligonucleotide(s) with said single-stranded nucleic acid, thereby obtaining a hybridization complex(es);
   (II) changing the temperature of the sample containing the hybridization complex(es) to dissociate the hybridization complex(es), and measuring the fluctuation of the fluorescence signal(s) due to the dissociation of the hybridization complex(es);
   (III) determining the Tm value(s), which is/are the dissociation temperature(s) of the hybridization complex(es), based on the fluctuation of said signal(s); and
   (IV) determining based on said Tm value(s) the presence of at least one polymorphism, or the abundance ratio(s) of a nucleic acid(s) having a polymorphism(s), which polymorphism(s) is/are at least one polymorphism selected from the group consisting of the polymorphism of the ALDH2 gene rs671 and the polymorphism of the ADH2 gene rs1229984.
(12) The method for detecting a polymorphism(s) according to (11), comprising amplifying the nucleic acid before said Step (I) or at the same time as said Step (I).
(13) A method comprising detecting at least one polymorphism selected from the group consisting of the polymorphism of the ALDH2 gene rs671 and the polymorphism of the ADH2 gene rs1229984, by the method for detecting a polymorphism(s) according to (10) to (12); and evaluating the capacity to metabolize, and/or judging tolerance to, alcohol based on the presence/absence of said polymorphism(s).
(14) A kit comprising the probe for detection of a polymorphism(s) according to any one of (1) to (9), which kit is used for detecting at least one polymorphism selected from the group consisting of genetic polymorphisms of the ALDH2 gene and genetic polymorphisms of the ADH2 gene. The invention also extends to the use of said kit for detecting said polymorphism(s).
(15) The kit for detection of a polymorphism(s) according to (14), further comprising a primer that enables amplification using as a template a region in the nucleotide sequence shown in SEQ ID NO:1 or 2 comprising a sequence with which said oligonucleotide (P1), (P1'), (P2) or (P2') hybridizes, and a primer that enables amplification using as a template a region in the nucleotide sequence shown in SEQ ID NO:13 comprising a sequence with which said oligonucleotide (P3) or (P3') hybridizes.
(16) A method using the probe according to (1) to (9), and primers which are used for detecting at least one polymorphism selected from the group consisting of the polymorphism of the ALDH2 gene rs671 and the polymorphism of the ADH2 gene rs1229984, said primers comprising the oligonucleotides (P4) and (P5) and/or (P6) and (P7) described below:
   (P4) an oligonucleotide comprising a nucleotide sequence of 21 to 60 consecutive nucleotides containing nucleotides 89 to 109 in SEQ ID NO:1 or 2;
   (P5) an oligonucleotide comprising a nucleotide sequence complementary to a nucleotide sequence of 20 to 60 consecutive nucleotides containing nucleotides 388 to 407 in SEQ ID NO:1 or 2;
   (P6) an oligonucleotide comprising a nucleotide sequence of 46 to 60 consecutive nucleotides containing nucleotides 93 to 138 in SEQ ID NO:13; and
   (P7) an oligonucleotide comprising a nucleotide sequence complementary to a nucleotide squence of 25 to 60 consecutive nucleotides containing nucleotides 214 to 238 in SEQ ID NO:13. The method of (10) or (11) comprising the use of said primers is also contemplated.
(17) A method for evaluating the capacity to metabolize, and/or judging tolerance to, alcohol, comprising evaluating the capacity to metabolize, and/or judging tolerance to, alcohol based on the presence/absence of a polymorphism(s), said method comprising detecting at least one polymorphism selected from the group consisting of the polymorphism of the ALDH2 gene rs671 and the polymorphism of the ADH2 gene rs1229984 by the method according to (16), and evaluating the capacity to metabolize, and/or judging tolerance to, alcohol based on the presence/absence of the polymorphism(s). Said method may be performed in a sample from a subject (e.g. a human) in whom the assessment is to be made.
(18) A reagent kit for detection of at least one polymorphism selected from the group consisting of the polymorphism of the ALDH2 gene rs671 and the polymorphism of the ADH2 gene rs1229984, comprising the probe according to any one of (1) to (9) and primers comprising the oligonucleotides (P4) and (P5) and/or (P6) and (P7) according to (16).

With the probes of the present invention, the polymorphism of the acetaldehyde dehydrogenase 2 (ALDH2) gene rs671 and the polymorphism of the alcohol dehydrogenase 2 (ADH2) gene rs1229984 can be detected clearly at the same time. In view of the clinical necessity to confirm gene mutations of ALDH2 and ADH2, being able to detect the two mutations at the same time is of significance.
By just adding the probes of the present invention and carrying out melting curve analysis (Tm analysis), the genetic polymorphisms of ALDH2 and ADH2 can be detected at the same time.
Since, by using the method of the present invention, the operation of recovery of an amplification product can be eliminated even in cases where PCR is carried out, there is hardly any risk of contamination. Further, since the operations in the method of the present invention are simple, they can be easily automated.
In the detection method of the present invention, a nucleic acid originally contained in unpurified saliva/whole blood can be used as a template.

### BRIEF DESCRIPTION OF THE DRAWINGS

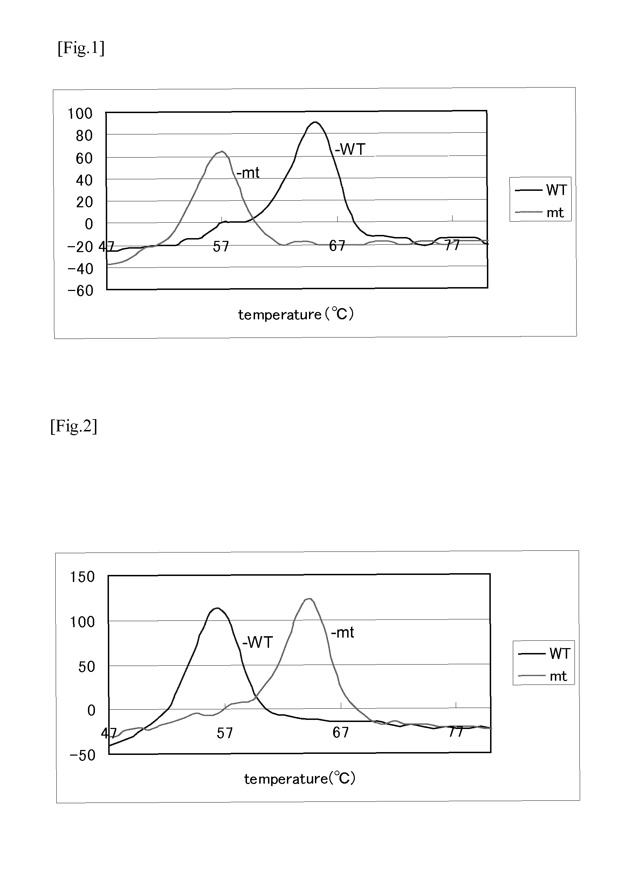

Fig. 1 shows the relationship between the amount of change in the fluorescence intensity of TAMRA per unit time (d the amount of increase in the fluorescence intensity/t) and the temperature in the Tm analysis in Example 1 in which 3T-ALDH2*2-wt-R1-21 was used as the probe, ALDH2*1 F50 (wild-type) and ALDH2*2 F50 (mutant) were used as the templates, and TAMRA was used as the fluorescent dye. The ordinate indicates the amount of change in the fluorescence intensity per unit time (d the amount of increase in the fluorescence intensity/t), and the abscissa indicates the temperature (°C). In the figures below, results obtained by the same experiments using different combinations of templates and a fluorescent dye are shown.
Fig. 2 shows the result obtained in Example 1 by using 3T-ALDH2*2-mt-F1-21 as the probe, ALDH2*1 R50 (wild-type) and ALDH2*2 R50 (mutant) as the templates, and TAMRA as the fluorescent dye.
Fig. 3 shows the result obtained in Example 1 by using 3T-ALDH2*2-wt-R3-20 as the probe, ALDH2*1 F50 (wild-type) and ALDH2*2 F50 (mutant) as the templates, and TAMRA as the fluorescent dye.
Fig. 4 shows the result obtained in Comparative Example 1 by using 5T-ALDH2*2-wt-R2-19 as the probe, ALDH2*1 F50 (wild-type) and ALDH2*2 F50 (mutant) as the templates, and TAMRA as the fluorescent dye.
Fig. 5A shows the result obtained in Example 2 by using 3FL-ADH2-WT-F3 as a probe, ADH2*1-R50 (wild-type) and ADH2*2-R50 (mutant) as the templates, and BODIPY FL as the fluorescent dye.
Fig. 5B shows a diagram in which the amount of change in fluorescence intensity in Fig. 5A is shown at an enlarged scale.
Fig. 6A shows the result obtained in Comparative Example 2 by using 3FL-ADH2-mt-F1 as the probe, ADH2*1-R50 (wild-type) and ADH2*2-R50 (mutant type) as the templates, and BODIPY FL as the fluorescent dye.
Fig. 6B shows a diagram in which the amount of change in fluorescence intensity in Fig. 6A is shown at an enlarged scale.
Fig. 7A shows the result obtained in Comparative Example 2 by using 3FL-ADH2-mt-F2 as the probe, ADH2*1-R51 (wild-type) and ADH2*2-R51 (mutant) as the templates, and BODIPY FL as the fluorescent dye.
Fig. 7B shows a diagram in which the amount of change in fluorescence intensity in Fig. 7A is shown at an enlarged scale.
Fig. 8A shows the result obtained in Example 3 by using 3T-ALDH2*2-mt-F1-21 as the probe, an oral swab as the template, and TAMRA as the fluorescent dye.
Fig. 8B shows the result obtained in Example 3 by using 3FL-ADH2-WT-F3 as the probe, an oral swab as the sample, and BODIPY FL as the fluorescent dye.
Fig. 9A shows the result obtained in Example 3 by using 3T-ALDH2*2-mt-F1-21 as the probe, whole blood as the sample, and TAMRA as the fluorescent dye.
Fig. 9B shows the result obtained in Example 3 by using 3FL-ADH2-WT-F3 as the probe, whole blood as the sample, and BODIPY FL as the fluorescent dye.
Fig. 10A shows the result obtained in Example 3 by using 3T-ALDH2*2-mt-F1-21 as the probe, purified DNA as the sample, and TAMRA as the fluorescent dye.
Fig. 10B shows the result obtained in Example 3 by using 3FL-ADH2-WT-F3 as the probe, purified DNA as the sample, and BODIPY FL as the fluorescent dye.

### MODE FOR CARRYING OUT THE INVENTION

### <1> Probe of Present Invention and Detection Method of Present Invention

The probe of the present invention is a probe for detection of at least 1 type of genetic polymorphism of the ALDH2 gene rs671 and the ADH2 gene rs1229984, comprising at least 1 type of fluorescently labeled oligonucleotide selected from (P1) to (P3') below:
(P1) an oligonucleotide comprising a nucleotide sequence complementary to a nucleotide sequence of 11 to 50 consecutive nucleotides containing nucleotides 241 to 251 in SEQ ID NO:1 or 2 or a homologous sequence thereof, wherein the nucleotide corresponding to the nucleotide at position 241 is a cytosine labeled with a fluorescent dye;
(P1') an oligonucleotide comprising a nucleotide sequence which hybridizes with a nucleotide sequence of 11 to 50 consecutive nucleotides containing nucleotides 241 to 251 in SEQ ID NO:1 or 2 under stringent conditions, wherein the nucleotide corresponding to the nucleotide at position 241 is a cytosine labeled with a fluorescent dye;
(P2) an oligonucleotide comprising a nucleotide sequence of 11 to 50 consecutive nucleotides containing nucleotides 251 to 261 in SEQ ID NO:1 or 2 or a homologous sequence thereof, wherein the nucleotide corresponding to the nucleotide at position 261 is a cytosine labeled with a fluorescent dye;
(P2') an oligonucleotide comprising a nucleotide sequence of 11 to 50 consecutive nucleotides containing nucleotides 251 to 261 in SEQ ID NO:1 or 2 or a nucleotide sequence which hybridizes with the complementary strand of SEQ ID NO: 1 or 2 under stringent conditions, wherein the nucleotide corresponding to the nucleotide at position 261 is a cytosine labeled with a fluorescent dye;
(P3) an oligonucleotide comprising a nucleotide sequence of 12 to 50 consecutive nucleotides containing nucleotides 201 to 212 in SEQ ID NO:13 or a homologous sequence thereof, wherein the nucleotide corresponding to the nucleotide at position 212 is a cytosine labeled with a fluorescent dye; and
(P3') an oligonucleotide comprising a nucleotide sequence of 12 to 50 consecutive nucleotides containing nucleotides 201 to 212 in SEQ ID NO:13 or a nucleotide sequence which hybridizes with the complementary strand of SEQ ID NO:13 under stringent conditions, wherein the nucleotide corresponding to the nucleotide at position 212 is a cytosine labeled with a fluorescent dye.
The polymorphism of the ALDH2 gene rs671 herein is located at nucleotide position 251 of SEQ ID NOs: 1 and 2. The polymorphism of the ADH2 gene rs1229984 is located at nucleotide position 201 of SEQ ID NOs:13 and 14. These rs numbers indicate registration numbers for the dbSNP database curated by the National Center for Biotechnology Information (//www.ncbi.nlm.nih.gov/projects/SNP/).

The probe of the present invention can be prepared in the same manner as the probes described in JP 2001-286300 A and JP 2002-119291 A except that the probe of the present invention has the above-described specified sequence in the nucleotide sequence shown in SEQ ID NO:1 (sequence having the wild-type nucleotide of the ALDH2 gene) or the nucleotide sequence shown in SEQ ID NO:2 (sequence having the mutant (polymorphic) nucleotide of the ALDH2 gene), and the above-described specified sequence in the nucleotide sequence shown in SEQ ID NO:13 (sequence having the wild-type nucleotide of the ADH2 gene).
The term "homologous sequence" herein means that a nucleotide sequence comprises a sequence having an identity of not less than 80%, 85%, 90%, 95%, 96%, 97%, 98% or 99% to a particular nucleotide sequence (e.g. SEQ ID NO:1, 2, or 13) or to the complementary strand of a particular nucleotide sequence. In the present invention, 100% identity may be included.
The hybridization herein can be carried out according to a known method or a method corresponding thereto, such as the method described in Molecular Cloning 3rd (J. Sambrook et al., Cold Spring Harbor Lab. Press, 2001). This literature is hereby incorporated in the present specification by reference. Preferably said hybridization is under stringent conditions.
The stringent conditions mean conditions under which a specific hybrid is formed while nonspecific hybrids are not formed. Typical examples of stringent conditions include conditions under which hybridization is performed with a potassium concentration of about 25 mM to about 50 mM and a magnesium concentration of about 1.0 mM to about 5.0 mM. Examples of the conditions in the present invention include conditions under which hybridization is performed in Tris-HCl (pH 8.6), 25 mM KCl and 1.5 mM MgCl₂, but the conditions are not limited thereto. Other examples of stringent conditions include those described in Molecular Cloning 3rd (J. Sambrook et al., Cold Spring Harbor Lab. Press, 2001). This literature is hereby incorporated in the present specification by reference. Those skilled in the art can easily select such conditions by controlling the hybridization reaction and/or changing the salt conditions of the hybridization reaction solution.
Oligonucleotides according to the above-mentioned fluorescently labeled oligonucleotides include modified oligonucleotides as well as oligonucleotides.
Examples of the nucleotides or units making up the oligonucleotides include ribonucleotides, deoxyribonucleotides, and artificial nucleic acids. The artificial nucleic acids include DNA; RNA; RNA analogue LNA (Locked Nucleic Acid); PNA (Peptide Nucleic Acid); BNA (Bridged Nucleic Acid) etc.
The above-mentioned oligonucleotides can be comprised of one or more kinds of the nucleotides or units.

The probes (P1) and (P1') of the present invention have a sequence complementary to a nucleotide sequence of 11 to 50 consecutive nucleotides containing nucleotides 241 to 251 in SEQ ID NO:1 or 2 or a homologous sequence thereof. That is, the sequence of the probes (P1) and (P1') of the present invention is complementary to a nucleotide sequence of 11 to 50 consecutive nucleotides containing nucleotides 241 to 251, but the sequence of the probe does not need to be completely complementary to such a nucleotide sequence. Further, the nucleotide corresponding to the nucleotide at position 241 is a cytosine and is labeled with a fluorescent dye.
The probes (P2) and (P2') of the present invention have a sequence of 11 to 50 consecutive nucleotides containing nucleotides 251 to 261 in SEQ ID NO:1 or 2 or a homologous sequence thereof. That is, the sequence of probes (P2) and (P2') of the present invention is homologous to a nucleotide sequence of 11 to 50 consecutive nucleotides containing nucleotides 251 to 261, but the sequence of the probe does not need to be completely identical to such a nucleotide sequence. Further, the nucleotide corresponding to the nucleotide at position 261 is a cytosine and is labeled with a fluorescent dye.
The probes (P3) and (P3') of the present invention have a sequence of 12 to 50 consecutive nucleotides containing nucleotides 201 to 212 in SEQ ID NO:13 or a homologous sequence thereof. That is, the sequence of probes (P3) and (P3') of the present invention is homologous to a nucleotide sequence of 12 to 50 consecutive nucleotides containing nucleotides 201 to 212, but the sequence of the probe does not need to be completely identical to such a nucleotide sequence. Further, the nucleotide corresponding to the nucleotide at position 212 is a cytosine and is labeled with a fluorescent dye.

The nucleotide length of each of the probes (P1) to (P3') of the present invention is 11 to 50, 12 to 30, 15 to 30, or 18 to 30, for example.
Examples of the nucleotide sequence of the probe to be used in the present invention for detecting the polymorphism of the ALDH2 gene rs671 include 5'-gttttcacttCagtgtatgcc-3' (SEQ ID NO:3), 5'-ggcatacactAaagtgaaaac-3' (SEQ ID NO:4) and 5'-ttttcacttTagtgtatgcc-3' (SEQ ID NO:5). Each nucleotide indicated by an uppercase letter represents the position of the mutation.
Examples of the nucleotide sequence of the probe for detecting the polymorphism of the ADH2 gene rs1229984 include 5'-TCTGTCNCACGGATGACC-3' (SEQ ID NO:15). In this sequence, "N" represents a, t, g or c. More particularly, the nucleotide sequence is 5'-TCTGTCGCACGGATGACC-3' (SEQ ID NO:16).
Examples of the fluorescent dye which may be used include those described in JP 2001-286300 A and JP 2002-119291 A, and specific examples of the fluorescent dye include Pacific Blue (trademark), FAM (trademark), TAMRA (trademark) and BODIPY (trademark) FL. Examples of the method for binding the fluorescent dye to the oligonucleotide include conventional methods such as the methods described in JP2001-286300A and JP2002-119291A.

For example, the probe of the present invention emits fluorescence from a fluorescent dye when the probe is not hybridized with the target sequence, and the fluorescence from the fluorescent dye decreases or increases when the probe is hybridized with the target sequence. For example, the probe of the present invention is a quenching probe which emits fluorescence from a fluorescent dye when the probe is not hybridized, and the fluorescence from the fluorescent dye is quenched when the probe is hybridized.
Further, the probe of the present invention has a nucleotide labeled with a fluorescent dye at the first, second or third position from the 3' end, or the probe is labeled with a fluorescent dye at the 3' end, for example.
The nucleotide labeled with a fluorescent dye in the probe of the present invention is the nucleotide at position 241 or 261 in SEQ ID NO:1 or 2. In SEQ ID NO:13 or 14, the nucleotide at position 212 is labeled.

The detection method of the present invention uses the probe of the present invention and comprises
(I) bringing the probe of the present invention for detecting a polymorphism into contact with a single-stranded nucleic acid in a sample, to allow hybridization of the fluorescently labeled oligonucleotide(s) with the single-stranded nucleic acid, thereby obtaining a hybridization complex(es);
(II) changing the temperature of the sample containing the hybridization complex(es) to dissociate the hybridization complex(es), and measuring the fluctuation of the fluorescence signal(s) due to the dissociation of the hybridization complex(es);
(III) determining the Tm value(s), which is/are the dissociation temperature(s) of the hybridization complex(es), based on the fluctuation of the signal(s); and
(IV) determining based on the Tm value(s) the presence of at least one polymorphism, or the abundance ratio(s) of a nucleic acid(s) having a polymorphism(s), which polymorphism(s) is/are at least one polymorphism selected from the group consisting of the polymorphism of the ALDH2 gene rs671 and the polymorphism of the ADH2 gene rs1229984.

The detection method of the present invention can be carried out according to conventional methods for nucleic acid amplification and melting curve analysis (Tm analysis) except that the probe of the present invention is used. Further, the detection method of the present invention may also comprise amplifying the nucleic acid before Step (I) or at the same time as Step (I).

The method of amplification preferably uses a polymerase, and examples of the method include PCR, ICAN and LAMP. When the amplification is carried out by a method using a polymerase, the amplification is preferably carried out in the presence of the probe of the present invention. Those skilled in the art can easily control reaction conditions and the like of the amplification depending on the probe to be used. By this, the detection can be carried out just by analyzing the Tm value of the probe after the amplification of the nucleic acid, so that the amplification product does not need to be subjected to purification and/or the like after the reaction. Therefore, there is no risk of contamination of the amplified product. Further, since the detection can be carried out with the same apparatus as the one necessary for the amplification, it is not necessary even to transfer the container. Therefore, automation can also be easily done.

In the present invention, the DNA in the sample may be either single-stranded DNA or double-stranded DNA. In cases where the DNA is double-stranded DNA, for example, the step of dissociating the double-stranded DNA in the sample by heating is preferably included before the hybridization step. By dissociating the double-stranded DNA into single-stranded DNA, hybridization with the detection probe is made possible in the subsequent hybridization step.

In the present invention, the ratio (molar ratio) of the probe of the present invention to be added with respect to the DNA in the sample is not restricted, and the ratio is, for example, not more than 1, or not more than 0.1 with respect to the DNA in the sample in view of to ensure a sufficient detection signal. In this case, for example, the DNA in the sample may be either the total amount of DNA having the polymorphism(s) to be detected and DNA which does not have the polymorphism(s) and should not be detected, or the total amount of an amplification product(s) containing a sequence(s) having the polymorphism(s) to be detected and amplification products containing sequences which do not have the polymorphism(s) to be detected and should not be detected. Although the ratio of the DNA to be detected relative to the DNA in the sample is usually not known, the ratio (molar ratio) of the probe to be added with respect to the DNA to be detected (the amplification product(s) containing the sequence(s) to be detected), as a result, is not more than 10, not more than 5, or not more than 3, for example. The lower limit of the ratio is not restricted, and the ratio is, for example, not less than 0.001, not less than 0.01, or not less than 0.1. The ratio of the probe of the present invention to be added with respect to the DNA may be, for example, either the molar ratio with respect to the double-stranded DNA or the molar ratio with respect to the single-stranded DNA.

The Tm value will now be described. Heating a solution containing double-stranded DNA causes an increase in the absorbance at 260 nm. This is caused because hydrogen bonds between the strands of the double-stranded DNA are broken by the heat and the double-stranded DNA is dissociated into single-stranded DNAs (melting of DNA). When all the double-stranded DNAs are dissociated into single-stranded DNAs, the absorbance becomes about 1.5 times as large as that observed when the heating was started (absorbance by only double-stranded DNA), and, by this, completion of the melting can be judged. Based on this phenomenon, the melting temperature Tm can be generally defined as the temperature at which the increase in the absorbance reached 50% of the total increase in the absorbance.

In the present invention, measurement of the signal fluctuation due to the temperature change for determination of the Tm value can be carried out also by measuring the absorbance at 260 nm based on the above-mentioned principle, but the measurement is preferably carried out based on a signal from a label added to the probe of the present invention, which signal fluctuates depending on the state of hybrid formation between the DNA and the probe. Therefore, as the probe of the present invention, the above-mentioned labeled probe is preferably used. Examples of the labeled probe include a fluorescently labeled oligonucleotide probe which emits fluorescence when it is not hybridized with the target sequence and whose fluorescence intensity decreases (quenching) when the probe is hybridized with the target sequence, and a fluorescently labeled oligonucleotide probe which emits fluorescence when it is not hybridized with the target sequence and whose fluorescence intensity increases when the probe is hybridized with the target sequence. In the case of the former probe, the probe shows no signal or a weak signal when it is forming a hybrid (double-stranded DNA) with the sequence to be detected, while the probe shows a signal or the signal increases when the probe is released by heating. In the case of the latter probe, the probe shows a signal by forming a hybrid (double-stranded DNA) with the sequence to be detected, while the signal decreases (disappears) when the probe is released by heating. Therefore, by detecting such a change in the signal due to the label under conditions (with the absorbance or the like) specific to the signal, determination of the progress of melting and the Tm value can be carried out in a similar manner to the measurement of the absorbance at 260 nm. For example, the labeling substance in the labeled probe is as mentioned above, and the probe is preferably a fluorescent dye-labeled probe.

The nucleic acid to be used as a template for carrying out the nucleic acid amplification is not restricted as long as it contains a nucleic acid, and examples of the nucleic acid include those derived from, or those which may be derived from, arbitrary biological origins such as blood; oral mucosal suspensions; somatic cells of nails, hair and the like; germ cells; milk; ascitic fluid; paraffin-embedded tissues; gastric juices; fluids obtained by gastric lavage; peritoneal fluid; amniotic fluid; and cell cultures. The nucleic acid as a template may be used as it is directly after being obtained from any of the above-described origins, or may be pretreated to modify properties of the sample before being used.

The method of nucleic acid amplification is further described by way of an example using PCR. The primer pair used in the PCR may be designed in the same manner as in the method for designing a primer pair for conventional PCR, except that the primer pair is designed such that a region with which the probe of the present invention can hybridize is amplified. The length and the Tm value of each primer is usually 12 mer to 40 mer and 40 to 70°C, or 16 mer to 30 mer and 55 to 65°C. The lengths of the primers of the primer pair may be different from each other, but the Tm values of both primers are preferably almost the same (the difference is usually not more than 2°C). The Tm value is a value calculated by the Nearest Neighbor method. Examples of the primer pair include those composed of primers having nucleotide sequences selected from SEQ ID NOs:7, 8, 19 and 20.

The PCR is preferably carried out in the presence of the probe of the present invention to be used in the present invention. By this, the Tm analysis can be carried out without subjecting the amplified product to purification and/or the like after the amplification reaction. Those skilled in the art can easily control the Tm values of the primers and the reaction conditions for the PCR (the composition of the reagent, number of cycles, and the like) depending on the probe used.

The Tm analysis can be carried out in the same manner as in a conventional method except that fluorescence from the fluorescent dye of the probe of the present invention is measured. The measurement of fluorescence can be carried out using an excitation light having a wavelength dependent on the fluorescent dye, to measure light having an emission wavelength dependent on the fluorescent dye. The heating rate in the Tm analysis is usually 0.1 to 1°C/second. The composition of the reaction solution used for carrying out the Tm analysis is not restricted as long as the probe can hybridize with a nucleic acid having the complementary sequence of the nucleotide sequence of the probe, and usually, the concentration of monovalent cations is 1.5 to 5 mM, and pH is 7 to 9. Since the reaction solution in an amplification method using a DNA polymerase, such as PCR, usually satisfies these conditions, the reaction solution after the amplification can be used as it is for the Tm analysis.

Detection of the polymorphism of the ALDH2 gene rs671 and the polymorphism of the ADH2 gene rs1229984 based on the result of the Tm analysis can be carried out according to a conventional method. The detection in the present invention includes detection of the presence/absence of a mutation and determination of the abundance of a nucleic acid having a polymorphism.

By the probe and the method for detecting a polymorphism of the present invention, polymorphisms in the ALDH2 gene and the ADH2 gene can be detected, and, based on the presence/absence of each polymorphism, the capacity to degrade/metabolize acetaldehyde or alcohol can be predicted. This prediction can be made in relation to a subject. More particularly, in cases where the nucleotides of the polymorphism of the ALDH2 gene rs671 are G/G, the gene is wild-type and hence is suggested to have a high capacity to degrade acetaldehyde, while in cases where the nucleotides are G/A, which is the heterozygote, or A/A, which is the homozygote of the mutant base, the capacity to degrade acetaldehyde is suggested to be low; and in cases where the nucleotides of the polymorphism of the ADH2 gene rs1229984 are G/G, the gene is wild-type and hence is suggested to have a low capacity to degrade alcohol, while in cases where the nucleotides are G/A, which is the heterozygote, or A/A, which is the homozygote of the mutant base, the capacity to degrade alcohol is suggested to be high.
Based on the result of prediction of the capacities to degrade acetaldehyde and alcohol, tolerance to alcohol and diseases related to the alcohol metabolism such as alcohol dependence, liver diseases and liver cancer can be predicted. Thus the probes of the present invention may used in the diagnosis of diseases related to the alcohol metabolism such as alcohol dependence, liver diseases and liver cancer (or for methods of diagnosis of said conditions using methods of the invention).
Further, in the method of the present invention, the polymorphism of the ALDH2 gene rs671 and the polymorphism of the ADH2 gene rs1229984 can be detected at the same time, and, for example, in cases where both the ALDH2 gene and the ADH2 gene have the mutations, it can be suggested that the tolerance to alcohol is lower.

### <2> Primers of Present Invention

The primers of the present invention are primers to be used in the detection method of the present invention together with the probe of the present invention.
More particularly, the primers of the present invention are primers for detecting at least one polymorphism selected from the group consisting of the polymorphism of the ALDH2 gene rs671 and the polymorphism of the ADH2 gene rs1229984, comprising the oligonucleotides (P4) and (P5) and/or (P6) and (P7) described below:
(P4) an oligonucleotide comprising a nucleotide sequence of 21 to 60 consecutive nucleotides containing nucleotides 89 to 109 in SEQ ID NO:1 or 2;
(P5) an oligonucleotide comprising a nucleotide sequence complementary to a nucleotide sequence of 20 to 60 consecutive nucleotides containing nucleotides 388 to 407 in SEQ ID NO:1 or 2;
(P6) an oligonucleotide comprising a nucleotide sequence of 46 to 60 consecutive nucleotides containing nucleotides 93 to 138 in SEQ ID NO:13; and
(P7) an oligonucleotide comprising a nucleotide sequence complementary to a nucleotide sequence of 25 to 60 consecutive nucleotides containing nucleotides 214 to 238 in SEQ ID NO:13.

The sequence of each of the primers (P4) to (P7) of the present invention does not need to be completely identical to the above-described sequence, and may be different by 5, 4, 3, 2 or 1 nucleotides. For example, the primers shown in SEQ ID NOs:7, 8, 19 and 20 may be used in the present invention.

### <3> Kit of Present Invention

The kit of the present invention is a kit to be used for the detection method of the present invention. This kit comprises the probe of the present invention for detecting a polymorphism. The kit of the present invention can also be used for judging the capacity to metabolize, and the tolerance to, alcohol.

The detection kit of the present invention may further comprise, in addition to the probe, reagents required for nucleic acid amplification in the detection method of the present invention, especially the above-described primers for amplification using a DNA polymerase.
In the detection kit of the present invention, the probe, primers and other reagents may be contained separately, or a mixture of a part of them may be contained. The present invention is described more concretely by way of Examples below. However, these Examples are merely examples and the present invention is not limited to the Examples.
In the present invention, in terms of the individual sequences in the sample nucleic acids, probes for detecting a polymorphism(s) and primers, matters described based on the complementary relationship between these are applied to the respective sequences and also to the sequences complementary thereto unless otherwise specified. When the matters of the present invention are applied to the sequence complementary to each sequence, the sequence recognized by the complementary sequence is read as the sequence complementary to the corresponding sequence described in the present specification throughout the specification according to the common technical knowledge.

### EXAMPLES

### Example 1 (Detection of Template Oligonucleotides for ALDH2 Using Single Probe)

Based on the nucleotide sequence comprising the site of the polymorphism of the ALDH2 gene rs671 (SEQ ID NO:1 (wild-type)), probes having C at the 3' end (which correspond to the wild-type (SEQ ID NOs:3 and 5) and the mutant (SEQ ID NO:4)) and a probe having C at the 5' end (which corresponds to the wild-type (SEQ ID NO:6)) shown in Table 1 were designed. In Table 1, the position of each probe is indicated by its nucleotides in the nucleotide sequence shown in SEQ ID NO:1 in the cases of the wild-type, and in the nucleotide sequence shown in SEQ ID NO:2 in the cases of the mutant. "P" at the 3' end indicates phosphorylation. Labeling with TAMRA was carried out according to a conventional method.
The sequences of the template oligonucleotides used as the subjects of detection (wild-type sense (SEQ ID NO:9), wild-type antisense (SEQ ID NO:11), mutant-type sense (SEQ ID NO:10) and mutant-type antisense (SEQ ID NO:12)) are shown in Table 1. In Table 1, the position of each oligonucleotide is indicated by its nucleotides in the nucleotide sequence shown in SEQ ID NO:2.

**[Table 1]**

| SEQ ID NO | Probe name | Sequence(5'→3') | Nucleotides |
|---|---|---|---|
| SEQ ID NO:3 | 3T-ALDH2*2-wt-R1-21 | gttttcacttCagtgtatgcc-(TAMRA) | 261-241 |
| SEQ ID NO:4 | 3T-ALDH2*2-mt-F1-21 | ggcatacactAaagtgaaaac-(TAMRA) | 241-261 |
| SEQ ID NO:5 | 3T-ALDH2*2-mt-R3-20 | ttttcacttTagtgtatgcc-(TAMRA) | 260-241 |
| SEQ ID NO:6 | 5T-ALDH2*2-wt-R2-19 | (TAMRA)-cttCagtgtatgcctgcag-P | 254-236 |

| | | | |
|---|---|---|---|
| *Nucleotides represented by uppercase letters indicate the position of mutation. | | | |

| SEQ ID NO | Template oligonucleotide name | Sequence(5'→3') | Nucleotides |
|---|---|---|---|
| SEQ ID NO:9 | ALDH2*1 F50 | gggcgagtacgggctgcaggcatacactGaagtgaaaactgtgagtgtgg | 223-272 |
| SEQ ID NO:10 | ALDH2*2 F50 | gggcgagtacgggctgcaggcatacactAaagtgaaaactgtgagtgtgg | 223-272 |
| SEQ ID NO:11 | ALDH2*1 R50 | ccacactcacagttttcacttCagtgtatgcctgcagcccgtactcgccc | 272-223 |
| SEQ ID NO:12 | ALDH2*2 R50 | ccacactcacagttttcacttTagtgtatgcctgcagcccgtactcgccc | 272-223 |

| | | | |
|---|---|---|---|
| *Nucleotides represented by uppercase letters indicate the position of mutation. | | | |

Tm analysis was carried out using Smart Cycler (manufactured by Cephied). The composition of the probe solution was as follows (Table 2). As samples, the following combinations of template oligonucleotides were used. The conditions of the Tm analysis were: 95°C for 1 second → 47°C for 60 seconds → (47°C → 94°C, 1°C/second).
The excitation wavelength and the detection wavelength in the Tm analysis were 520 to 555 nm and 585 to 700 nm (TAMRA), respectively.

**[Table 2]**

| Formulation | | Total 25µl |
|---|---|---|
| | 1 ×GeneTaq Buffer | |
| | Probe | 0.2µM |
| | Template oligonucleotide (WT mt)* | 0.4µM |

| | | |
|---|---|---|
| *The template oligonucleotide was prepared by mixing equal amounts of the following WT and mt oligonucleotides before use. | | |

| Probe | Template oligonucleotide (WT) | Template oligonucleotide (mt) |
|---|---|---|
| 3T-ALDH2*2-wt-R1-21 (SEQ ID NO:3) | ALDH2*1 F50 (SEQ ID NO:9) | ALDH2*2 F50 (SEQ ID NO:10) |
| 3T-ALDH2*2-mt-F1-21(SEQ ID NO:4) | ALDH2*1 R50 (SEQ ID NO:11) | ALDH2*2 R50 (SEQ ID NO:12) |
| 3T-ALDH2*2-mt-R3-20 (SEQ ID NO:5) | ALDH2*1 F50 (SEQ ID NO:9) | ALDH2*2 F50 (SEQ ID NO:10) |
| 5T-ALDH2*2-wt-R2-19 (SEQ ID NO:6) | ALDH2*1 F50 (SEQ ID NO:9) | ALDH2*2 F50 (SEQ ID NO:10) |

| | | |
|---|---|---|
| *Combinations of the probe and the template oligonucleotide | | |

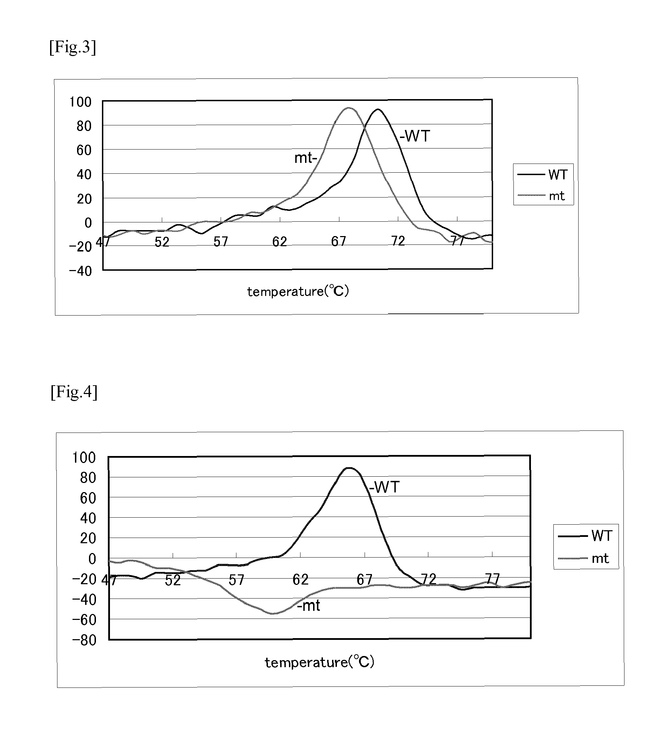

As a result of Tm analysis using the probes shown in Table 1, two clear peaks, that is, a peak of TAMRA corresponding to the wild-type and a peak of TAMRA corresponding to the mutant were observed with 3T-ALDH2*2-wt-R1-21 (SEQ ID NO:3), 3T-ALDH2*2-mt-F1-21 (SEQ ID NO:4) and 3T-ALDH2*2-mt-R3-20 (SEQ ID NO:5) (Figs. 1 to 3), but a peak of TAMRA corresponding to the mutant was not observed with 5T-ALDH2*2-wt-R2-19 (SEQ ID NO:6) (Fig. 4).
Therefore, it can be understood that, even in cases where the probe is labeled at C located at its 5' or 3' end, the probe sequence cannot be arbitrary, and that it is important for the probe to be fluorescently labeled at C located at position 241 or 261, as in the case of the probes having the sequences shown in SEQ ID NOs:3 to 5.

### Example 2 (Detection of Template Oligonucleotides for ADH2 Using Single Probe)

Based on the nucleotide sequence comprising the site of the polymorphism of the ADH2 gene rs1229984 (SEQ ID NO:13 (wild-type)), probes having C at an end (which correspond to the wild-type (SEQ ID NO:16) and the mutant (SEQ ID NOs:17 and 18)) shown in Table 3 were designed. In Table 3, the position of each probe is indicated by its nucleotides in the nucleotide sequence shown in SEQ ID NO:13 in the cases of the wild-type, and in the nucleotide sequence shown in SEQ ID NO:14 in the cases of the mutant. Labeling with BODIPY FL was carried out according to a conventional method.
Further, the sequences of the template oligonucleotides used as the subjects of detection (which correspond to the wild-type (SEQ ID NOs:21 and 23) and the mutant (SEQ ID NOs:22 and 24)) are shown in Table 3. In Table 3, the position of each oligonucleotide is indicated by its nucleotides in the nucleotide sequence shown in SEQ ID NO:13 or 14.
In SEQ ID NOs:13 and 14, w represents a or t, and k represents g or t.

**[Table 3]**

| SEQ ID NO | Probe name | Sequence(5'→3') | Nucleotides |
|---|---|---|---|
| SEQ ID NO:16 | 3FL-ADH2-WT-F3 | tctgtcGcacggatgacc-(BODIPY-FL) | 195-212 |
| SEQ ID NO:17 | 3FL-ADH2-mt-F1 | tctgtcAcacagatgacc-(BODIPY-FL) | 195-212 |
| SEQ ID NO:18 | 3FL-ADH2-mt-F2 | tgtcAcacagatgaccac-(BODIPY-FL) | 197-214 |

| | | | |
|---|---|---|---|
| *Nucleotides represented by uppercase letters indicate the position of mutation. | | | |

| SEQ ID NO | Template oligonucleotide name | Sequence(5'→3') | Nucleoti des |
|---|---|---|---|
| SEQ ID NO:21 | ADH2*1-F50 | ggtggctgtaggaatctgtcGcacagatgaccacgtggttagtggcaacc | 181-230 |
| SEQ ID NO:22 | ADH2*2-F50 | ggtggctgtaggaatctgtcAcacagatgaccacgtggttagtggcaacc | 181-230 |
| SEQ ID NO:23 | ADH2*1-R50 | ggttgccactaaccacgtggtcatctgtgCgacagattcctacagccacc | 230-181 |
| SEQ ID NO:24 | ADH2*2-R50 | ggttgccactaaccacgtggtcatctgtgTgacagattcctacagccacc | 230-181 |

| | | | |
|---|---|---|---|
| *Nucleotides represented by uppercase letters indicate the position of mutation. | | | |

Tm analysis was carried out using a fully automatic SNPs testing device (trade name: i-densy IS-5310, manufactured by ARKRAY, Inc.). The composition of the PCR reaction solution was as follows (Table 4). As samples, the following combinations of template oligonucleotides were used. The conditions of the Tm analysis were: 95°C for 1 second → 40°C for 60 seconds → (40°C → 66°C, 3°C/second).
The excitation wavelength and the detection wavelength in the Tm analysis were 420 to 485 nm and 520 to 555 nm (BODIPY FL), respectively.

**[Table 4]**

| Formulation ) | | Total 25µL |
|---|---|---|
| | 1×GeneTaq Buffer | |
| | Probe | 0.2µM |
| | Template oligonucleotide(WTmt)* | 0.4µM |

| | | |
|---|---|---|
| *The template oligonucleotide was prepared by mixing equal amounts of the following WT and mt oligonucleotides before use. | | |

| Probe | Template (WT) | Template (mt) |
|---|---|---|
| 3FL-ADH2-WT-F3 (SEQ ID NO:16) | ADH2*1-R50(SEQ ID NO:23) | ADH2*2-R50(SEQ ID NO:24) |
| 3FL-ADH2-mt-F1 (SEQ ID NO:17) | ADH2*1-R50(SEQ ID NO:23) | ADH2*2-R50(SEQ ID NO:24) |
| 3FL-ADH2-mt-F2(SEQ ID NO:18) | ADH2*1-R50(SEQ ID NO:21) | ADH2*2-R50(SEQ ID NO:22) |

| | | |
|---|---|---|
| 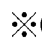 Combinations of the probe and the template oligonucleotide | | |

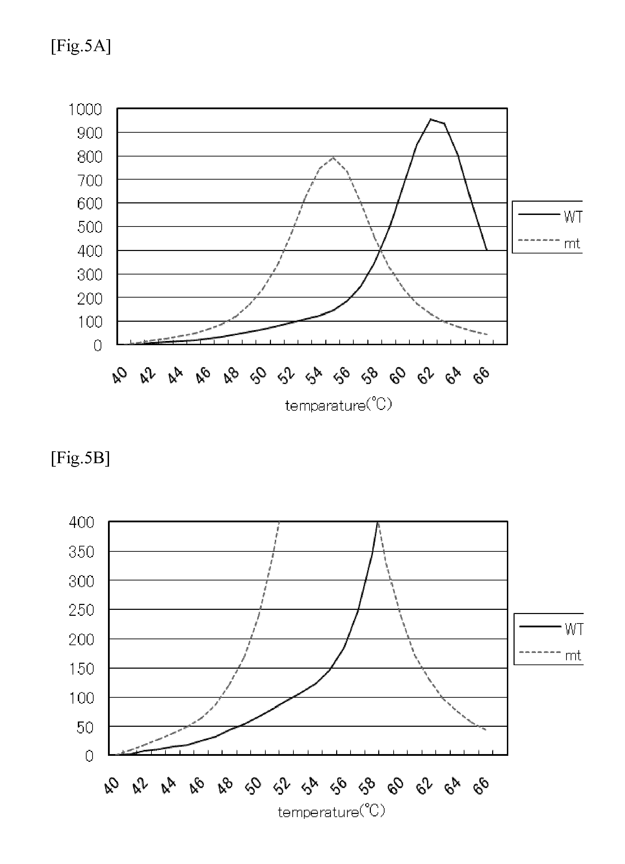

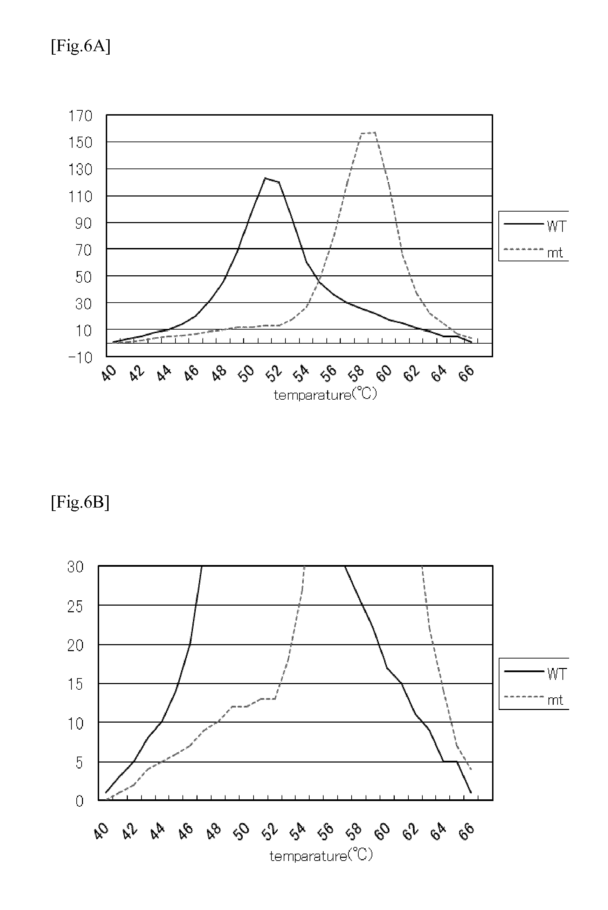

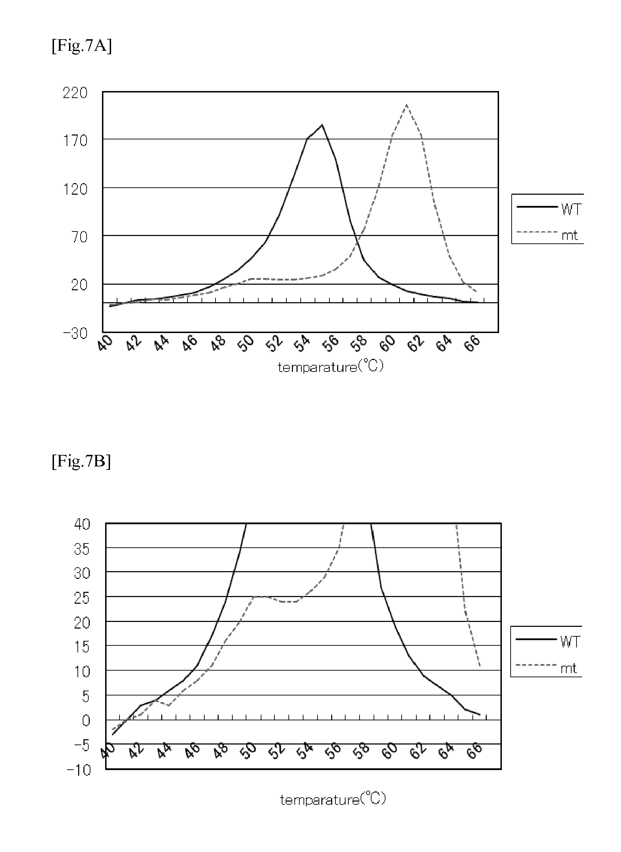

As a result of Tm analysis using the probes shown in Table 3, two clear peaks, that is, a peak of BODIPY FL corresponding to the wild-type and a peak of BODIPY FL corresponding to the mutant were observed with 3FL-ADH2-WT-F3 (SEQ ID NO:16) (Fig. 5), but a peak of BODIPY FL corresponding to the mutant - was not observed with 3FL-ADH2-mt-F1 (SEQ ID NO:17) and 3FL-ADH2-mt-F2 (SEQ ID NO:18) (Figs. 6A and 7A).
In Fig. 6B, the mt graph shows noise at 50°C and a peak at 59°C, and, in such a case, the genotype may be erroneously determined to be heterozygous. Further, in Fig. 7B, the mt graph shows noise at 51°C and a peak at 61 °C, and, in such a case, the genotype may be erroneously determined to be heterozygous.
Therefore, it can be understood that, even in cases where the probe is labeled at C located at its 3' end, the probe sequence cannot be arbitrary, and that it is important for the probe to be fluorescently labeled at C located at position 212 and to have a wild-type sequence, as in the case of the probe having the sequence shown in SEQ ID NO:16.

### Example 3 (Detection of Oral Swab, Whole Blood or Purified DNA Using Plurality of Probes)

As described below, the following primers were used to amplify the polymorphic region from an oral swab, whole blood or purified DNA by PCR, and Tm analysis was carried out using the probes having the sequences shown in SEQ ID NOs:4 and 16.
First, based on the nucleotide sequence comprising the site of the polymorphism of the ALDH2 gene rs671 (SEQ ID NO:1 (wild-type)), the primers shown in Table 5 were designed such that the polymorphic site can be amplified. In Table 5, the position of each primer is indicated by its nucleotides in the nucleotide sequence shown in SEQ ID NO:1.
Further, based on the nucleotide sequence comprising the site of the polymorphism of the ADH2 gene rs1229984 (SEQ ID NO:13 (wild-type)), the primers shown in Table 5 were designed such that the polymorphic site can be amplified. In Table 5, the position of each primer is indicated by its nucleotides in the nucleotide sequence shown in SEQ ID NO:13.
Subsequently, PCR and Tm analysis were carried out using a fully automatic SNPs testing device (trade name: i-densy IS-5310, manufactured by ARKRAY, Inc.). The composition of the PCR reaction solution was as follows below. As a sample, the following oral swab, whole blood or purified DNA was used. The conditions of the PCR and Tm analysis were: 95°C for 60 seconds → (95°C for 1 second → 60°C for 30 seconds) × 50 cycles → 95°C for 1 second → 40°C for 60 seconds → (40°C → 75°C, 1°C/3 seconds).
The excitation wavelength and the detection wavelength in the Tm analysis were 420 to 485 nm and 520 to 555 nm (BODIPY FL), respectively, or 520 to 555 nm and 585 to 700 nm (TAMRA).

**[Table 5]**

| SEQ ID NO | Primer name | Sequence(5'→3') | Nucleotides |
|---|---|---|---|
| SEQ ID NO:7 | ALDH2 F4 | Ctgggagtgtaacccataacc | 89-109 |
| SEQ ID NO:8 | ALDH2 R9 900-881 | Cagcaggccctgagtccccg | 407-388 |
| SEQ ID NO:19 | ADH2-F3 | gaaacacaatttcaggaatttgggtatgttaaattcatctagttac | 93-138 |
| SEQ ID NO:20 | ADH2-R4 | Ggwcaccagkttgccactaaccacg | 238-214 |

| | | | |
|---|---|---|---|
| *In SEQ ID NO:20, w represents a or t, and k represents g or t. | | | |

| (Reaction solution volume: 50µl) | |
|---|---|
| 1 × PCR buffer | |
| dNTP | 0.2mM |
| MgCl₂ | 1.5mM |
| Taq polymerase (manufactured by ARKRAY, Inc.) | 0.0376U |
| 100µM ALDH2 F4 | 0.5µM |
| 100µM ALDH2 R9 | 1µM |
| 100µM ADH2 F3 | 0.5µM |
| 100µM ADH2 R4 | 1µM |
| 3T-ALDH2*2-mt-F1-21(SEQ ID NO:4) | 0.2µM |
| 3FL-ADH2-WT-F3(SEQ ID NO:16) | 0.1µM |
| Sample | 4µl |

### <Preparation of Oral Swab>

In 500 µl of an oral swab-suspending solution (1), an oral swab collected by rubbing over both cheeks 20 times was suspended. To 90 µl of a diluent (2), 30 µl of the resulting suspension were added, and the resulting mixture was mixed well. A 10-µl aliquot of the mixture was then heated at 95°C for 5 minutes, to obtain 4 µl of a pretreated oral swab liquid. The resulting liquid was added to the PCR reaction solution, and DNA derived from the pretreated oral swab liquid was used as a template.

**[Table 6]**

| | |
|---|---|
| Oral swab-suspending solution (1) | ×1 |
| Tris-HCl (pH8.0) | 10mM |
| EDTA (pH8.0) | 0.1mM |
| SDS | 0.30% |
| NaN₃ | 0.05% |
| ProteinaseK | 0.25µg/µl |
| Diluent (2) | |
| Tris-HCl (pH8.0) | 10mM |
| EDTA (pH8.0) | 0.1mM |

### <Preparation of Whole Blood>

In 90 µl of a diluent (1), 10 µl of whole blood were added, and the resulting mixture was mixed well, followed by adding 10 µl of this mixture to 90 µl of a diluent (2). A 17-µl aliquot of the resulting mixture was then heated at 95°C for 10 minutes, to obtain 4 µl of pretreated whole blood. This was added to the PCR reaction solution, and DNA derived from the pretreated whole blood was used as a template.

**[Table 7]**

| Diluent(1) | |
|---|---|
| Tris-HCl (pH8.0) | 10mM |
| EDTA (pH8.0) | 0.1mM |
| SDS | 0.30% |

| Diluent(2) | |
|---|---|
| Tris-HCl (pH8.0) | 10mM |
| 500mM EDTA (pH8.0) | 0.1mM |

### <Purified DNA>

In each test, 4 µl of purified DNA at a concentration of 25 copies/ µl was added to the PCR reaction solution, as a template.

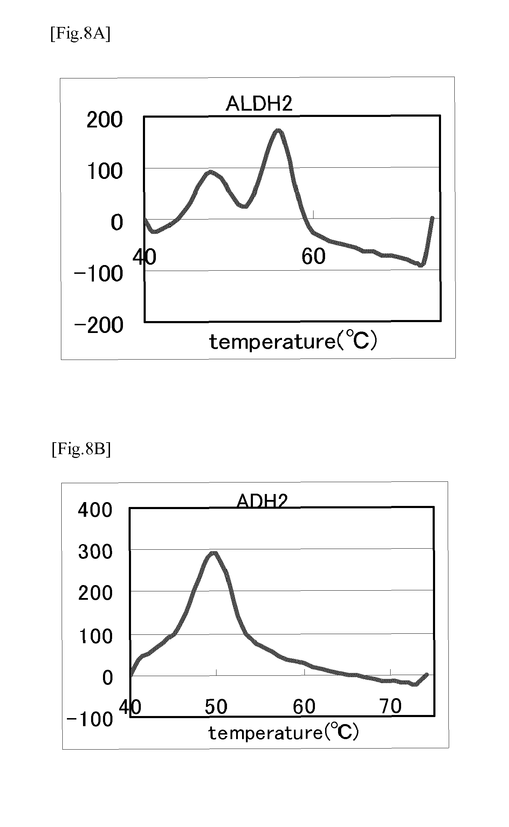

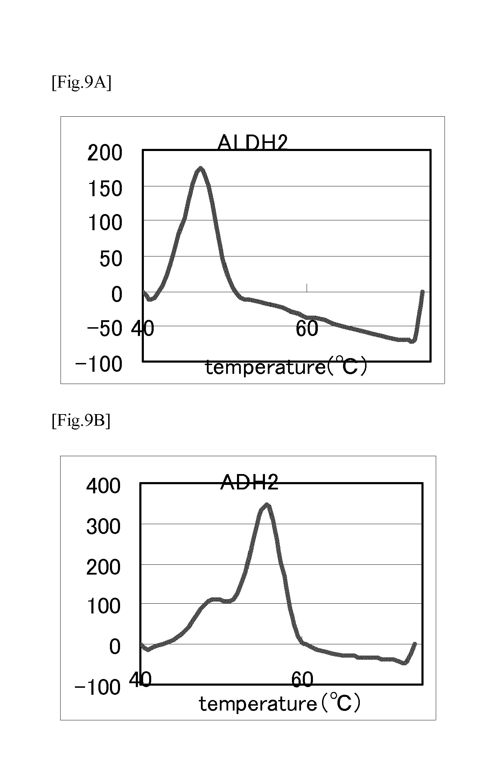

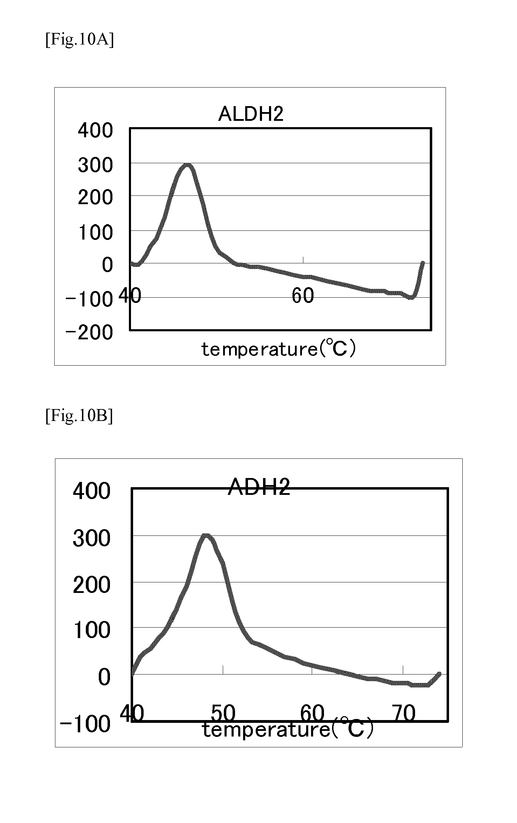

As a result of evaluation of the ALDH2 gene based on fluorescence of TAMRA, the heterozygous oral swab showed a peak of TAMRA fluorescence corresponding to the wild-type and a peak of TAMRA fluorescence corresponding to the mutant (Fig. 8A), while the homozygous whole blood and DNA showed only a peak of TAMRA fluorescence corresponding to the mutant (Fig. 9A and 10A).
Further, as a result of evaluation of the ADH2 gene based on fluorescence of BODIPY FL, the wild-type oral swab and DNA showed only a peak of BODIPY FL fluorescence corresponding to the wild-type (Figs. 8B and 10B), and the heterozygous whole blood showed a peak of BODIPY FL fluorescence corresponding to the wild-type and a peak of BODIPY FL fluorescence corresponding to the mutant (Fig. 9B).

Based on the results shown in Figs. 8 to 10, changes in fluorescence intensity which can be analyzed by Tm analysis were observed for the polymorphism of the ALDH2 gene and the polymorphism of the ADH2 gene when the probe having the sequence shown in SEQ ID NO:4 in Table 1 and the probe having the sequence shown in SEQ ID NO:16 in Table 3 were used.
That is, in the cases of the polymorphism of the ALDH2 gene, the oral swab with G/A, which is the heterozygote, showed 2 peaks (48°C and 56°C), while the whole blood and DNA with A/A, which is a homozygote, showed only 1 peak (48°C), so that a unique change in the pattern of the amount of change in fluorescence intensity exists.
Further, in the cases of the polymorphism of the ADH2 gene, a change in the fluorescence intensity which can be analyzed by Tm analysis was also observed. That is, the whole blood with G/A, which is the heterozygote, showed 2 peaks (50°C and 56°C), while the oral swab and DNA with G/G, which is the wild-type homozygote, showed only 1 peak (50°C), so that a unique change in the pattern of the amount of change in the fluorescence intensity exists.
Therefore, by using the probes having the sequences shown in SEQ ID NOs:4 and 16 at the same time, the polymorphism of the ALDH2 gene and the polymorphism of the ADH2 gene can be detected at the same time.
Further, since the probes having the sequences shown in SEQ ID NOs:3 and 5 are fluorescently labeled at C located at the 3' end as in the case of the probe having the sequence of SEQ ID NO:4, and the effect of those probes were demonstrated in Example 1, the polymorphism of the ALDH2 gene and the polymorphism of the ADH2 gene can be detected at the same time also by using the probe having the sequence shown in SEQ ID NO:3 or 5 and the probe having the sequence shown in SEQ ID NO:16 at the same time.

### INDUSTRIAL APPLICABILITY

By using the probe of the present invention, the capacity of a subject to biologically degrade alcohol and the tolerance of the subject to alcohol can be predicted.

## Claims

1. A probe for detection of at least 1 type of genetic polymorphism of the ALDH2 gene rs671 and the ADH2 gene rs1229984, comprising at least 1 type of fluorescently labeled oligonucleotide selected from (P1) to (P3') below:
(P1) an oligonucleotide comprising a nucleotide sequence complementary to a nucleotide sequence of 11 to 50 consecutive nucleotides containing nucleotides 241 to 251 in SEQ ID NO:1 or 2 or a homologous sequence thereof, wherein the nucleotide corresponding to the nucleotide at position 241 is a cytosine labeled with a fluorescent dye;
(P1') an oligonucleotide comprising a nucleotide sequence which hybridizes with a nucleotide sequence of 11 to 50 consecutive nucleotides containing nucleotides 241 to 251 in SEQ ID NO:1 or 2 under stringent conditions, wherein the nucleotide corresponding to the nucleotide at position 241 is a cytosine labeled with a fluorescent dye;
(P2) an oligonucleotide comprising a nucleotide sequence of 11 to 50 consecutive nucleotides containing nucleotides 251 to 261 in SEQ ID NO:1 or 2 or a homologous sequence thereof, wherein the nucleotide corresponding to the nucleotide at position 261 is a cytosine labeled with a fluorescent dye;
(P2') an oligonucleotide comprising a nucleotide sequence of 11 to 50 consecutive nucleotides containing nucleotides 251 to 261 in SEQ ID NO:1 or 2 or a nucleotide sequence which hybridizes with the complementary strand of SEQ ID NO: 1 or 2 under stringent conditions, wherein the nucleotide corresponding to the nucleotide at position 261 is a cytosine labeled with a fluorescent dye;
(P3) an oligonucleotide comprising a nucleotide sequence of 12 to 50 consecutive nucleotides containing nucleotides 201 to 212 in SEQ ID NO:13 or a homologous sequence thereof, wherein the nucleotide corresponding to the nucleotide at position 212 is a cytosine labeled with a fluorescent dye; and
(P3') an oligonucleotide comprising a nucleotide sequence of 12 to 50 consecutive nucleotides containing nucleotides 201 to 212 in SEQ ID NO:13 or a nucleotide sequence which hybridizes with the complementary strand of SEQ ID NO:13 under stringent conditions, wherein the nucleotide corresponding to the nucleotide at position 212 is a cytosine labeled with a fluorescent dye.

2. The probe according to claim 1, wherein
said oligonucleotides (P1) and (P1') have the nucleotide corresponding to the nucleotide at position 241 labeled with a fluorescent dye at the first, second or third position from the 3' end;
said oligonucleotides (P2) and (P2') have the nucleotide corresponding to the nucleotide at position 261 labeled with a fluorescent dye at the first, second or third position from the 3' end; and
said oligonucleotides (P3) and (P3') have the nucleotide corresponding to the nucleotide at position 212 labeled with a fluorescent dye at the first, second or third position from the 3' end.

3. The probe according to claim 1, wherein
said oligonucleotides (P1) and (P1') have the nucleotide corresponding to the nucleotide at position 241 labeled with a fluorescent dye at the 3' end;
said oligonucleotides (P2) and (P2') have the nucleotide corresponding to the nucleotide at position 261 labeled with a fluorescent dye at the 3' end; and
said oligonucleotides (P3) and (P3') have the nucleotide corresponding to the nucleotide at position 212 labeled with a fluorescent dye at the 3' end.

4. The probe for detecting a polymorphism according to any one of claims 1 to 3, wherein said oligonucleotide emits fluorescence when the oligonucleotide is not hybridized with a target sequence, and the fluorescence intensity decreases or increases when the oligonucleotide is hybridized with the target sequence, preferably wherein the fluorescence intensity decreases when said oligonucleotide is hybridized with the target sequence.

5. The probe according to any one of claims 1 to 4, wherein said oligonucleotides (P1) to (P3') have 12 to 30 consecutive nucleotides, preferably 15 to 30 consecutive nucleotides, further preferably 18 to 30 consecutive nucleotides.

6. The probe according to any one of claims 1 to 5, wherein said probe is a probe for melting curve analysis.

7. A method for detecting at least one polymorphism selected from the group consisting of the polymorphism of the ALDH2 gene rs671 and the polymorphism of the ADH2 gene rs1229984 in a sample containing nucleic acid which may contain said polymorphism comprising contacting at least one probe according to any one of claims 1 to 6 with said sample and detecting the presence or absence of said polymorphism(s).

8. The method for detecting a polymorphism(s) according to claim 7, comprising
(I) bringing the probe for detection of a polymorphism(s) according to any one of claims 1 to 6 into contact with a single-stranded nucleic acid in said sample, to allow hybridization of said fluorescently labeled oligonucleotide(s) with said single-stranded nucleic acid, thereby obtaining a hybridization complex(es);
(II) changing the temperature of the sample containing the hybridization complex(es) to dissociate the hybridization complex(es), and measuring the fluctuation of the fluorescence signal(s) due to the dissociation of the hybridization complex(es);
(III) determining the Tm value(s), which is/are the dissociation temperature(s) of the hybridization complex(es), based on the fluctuation of said signal(s); and
(IV) determining based on said Tm value(s) the presence of at least one polymorphism, or the abundance ratio(s) of a nucleic acid(s) having a polymorphism(s), which polymorphism(s) is/are at least one polymorphism selected from the group consisting of the polymorphism of the ALDH2 gene rs671 and the polymorphism of the ADH2 gene rs1229984,
wherein preferably the nucleic acid is amplified before said Step (I) or at the same time as said Step (I).

9. A kit comprising the probe for detection of a polymorphism(s) according to any one of claims 1 to 6, which kit is used for detecting at least one polymorphism selected from the group consisting of genetic polymorphisms of the ALDH2 gene and genetic polymorphisms of the ADH2 gene.

10. The kit for detection of a polymorphism(s) according to claim 9, further comprising a primer that enables amplification using as a template a region in the nucleotide sequence shown in SEQ ID NO:1 or 2 comprising a sequence with which said oligonucleotide (P1), (P1'), (P2) or (P2') hybridizes, and a primer that enables amplification using as a template a region in the nucleotide sequence shown in SEQ ID NO:13 comprising a sequence with which said oligonucleotide (P3) or (P3') hybridizes.

11. Use of a kit according to claim 9 or 10 for detecting at least one polymorphism selected from the group consisting of genetic polymorphisms of the ALDH2 gene and genetic polymorphisms of the ADH2 gene.

12. A method according to claim 7 or 8, wherein said method comprises use of primers for detecting said polymorphism, said primers comprising the oligonucleotides (P4) and (P5) and/or (P6) and (P7) described below:
(P4) an oligonucleotide comprising a nucleotide sequence of 21 to 60 consecutive nucleotides containing nucleotides 89 to 109 in SEQ ID NO:1 or 2;
(P5) an oligonucleotide comprising a nucleotide sequence complementary to a nucleotide sequence of 20 to 60 consecutive nucleotides containing nucleotides 388 to 407 in SEQ ID NO:1 or 2;
(P6) an oligonucleotide comprising a nucleotide sequence of 46 to 60 consecutive nucleotides containing nucleotides 93 to 138 in SEQ ID NO:13; and
(P7) an oligonucleotide comprising a nucleotide sequence complementary to a nucleotide sequence of 25 to 60 consecutive nucleotides containing nucleotides 214 to 238 in SEQ ID NO:13.

13. A method for evaluating capacity to metabolize, and/or judging tolerance to, alcohol, comprising evaluating capacity to metabolize, and/or judging tolerance to, alcohol based on the presence/absence of a polymorphism(s) in a subject, said method comprising detecting at least one polymorphism selected from the group consisting of the polymorphism of the ALDH2 gene rs671 and the polymorphism of the ADH2 gene rs1229984 in a sample from said subject by the method according to claim 7, 8 or 12, and evaluating capacity to metabolize, and/or judging tolerance to, alcohol based on the presence/absence of the polymorphism(s).

14. The kit of claim 9 or 10 further comprising the primers (P4) and (P5) and/or (P6) and (P7) as defined in claim 12.

15. The probe according to claims 1 to 6 for use in the diagnosis of diseases related to the alcohol metabolism such as alcohol dependence, liver diseases and liver cancer.


SEQUENCE LISTING

<110>  ARKRAY,INC.

<120>  Method for Simultaneously Detecting Gene Mutations of Acetaldehyde
Dehydrogenase 2 and Alcohol Dehydrogenase 2

<130>  42.13.112769

<150>  JP2011-102333
<151>  2011-04-28

<160>  24

<170>  PatentIn version 3.5

<210>  1
<211>  501
<212>  DNA
<213>  Homo sapiens

<400>  1
gggcaacaga gaaagattct atctcaaaaa aaaaaatttt tttttaagtt aaaaataaaa      60

taaagacttt ggggcaatac agggggtcct gggagtgtaa cccataaccc ccaagagtga      120

tttctgcaat ctcgtttcaa attacagggt caactgctat gatgtgtttg gagcccagtc      180

accctttggt ggctacaaga tgtcggggag tggccgggag ttgggcgagt acgggctgca      240

ggcatacact gaagtgaaaa ctgtgagtgt gggacctgct gggggctcag ggcctgttgg      300

ggcttgaggg tctgctggtg gctcggagcc tgctggggga ttggggtctg ttgggggctc      360

ggggcctgcc agaggttcag gacctgccgg ggactcaggg cctgctggaa gttcaggacc      420

tgctggggat cagggcctgc cagggattta gggtctgctg ggcgggccac cttttggcct      480

ctccctcatg cttgaggcca t                                                501


<210>  2
<211>  501
<212>  DNA
<213>  Homo sapiens

<400>  2
gggcaacaga gaaagattct atctcaaaaa aaaaaatttt tttttaagtt aaaaataaaa      60

taaagacttt ggggcaatac agggggtcct gggagtgtaa cccataaccc ccaagagtga      120

tttctgcaat ctcgtttcaa attacagggt caactgctat gatgtgtttg gagcccagtc      180

accctttggt ggctacaaga tgtcggggag tggccgggag ttgggcgagt acgggctgca      240

ggcatacact aaagtgaaaa ctgtgagtgt gggacctgct gggggctcag ggcctgttgg      300

ggcttgaggg tctgctggtg gctcggagcc tgctggggga ttggggtctg ttgggggctc      360

ggggcctgcc agaggttcag gacctgccgg ggactcaggg cctgctggaa gttcaggacc      420

tgctggggat cagggcctgc cagggattta gggtctgctg ggcgggccac cttttggcct      480

ctccctcatg cttgaggcca t                                                501

<210>  3
<211>  21
<212>  DNA
<213>  Artificial Sequence

<220>
<223>  probe

<400>  3
gttttcactt cagtgtatgc c                                                21


<210>  4
<211>  21
<212>  DNA
<213>  Artificial Sequence

<220>
<223>  probe

<400>  4
ggcatacact aaagtgaaaa c                                                21


<210>  5
<211>  20
<212>  DNA
<213>  Artificial Sequence

<220>
<223>  probe

<400>  5
ttttcacttt agtgtatgcc                                                  20


<210>  6
<211>  19
<212>  DNA
<213>  Artificial Sequence

<220>
<223>  probe

<400>  6
cttcagtgta tgcctgcag                                                   19


<210>  7
<211>  21
<212>  DNA
<213>  Artificial Sequence

<220>
<223>  primer

<400>  7
ctgggagtgt aacccataac c                                                21


<210>  8
<211>  20
<212>  DNA
<213>  Artificial Sequence

<220>
<223>  primer

<400>  8
cagcaggccc tgagtccccg                                                    20


<210>  9
<211>  50
<212>  DNA
<213>  Artificial Sequence

<220>
<223>  oligonucleotide

<400>  9
gggcgagtac gggctgcagg catacactga agtgaaaact gtgagtgtgg                   50


<210>  10
<211>  50
<212>  DNA
<213>  Artificial Sequence

<220>
<223>  oligonucleotide

<400>  10
gggcgagtac gggctgcagg catacactaa agtgaaaact gtgagtgtgg                   50


<210>  11
<211>  50
<212>  DNA
<213>  Artificial Sequence

<220>
<223>  oligonucleotide

<400>  11
ccacactcac agttttcact tcagtgtatg cctgcagccc gtactcgccc                   50


<210>  12
<211>  50
<212>  DNA
<213>  Artificial Sequence

<220>
<223>  oligonucleotide

<400>  12
ccacactcac agttttcact ttagtgtatg cctgcagccc gtactcgccc                   50


<210>  13
<211>  501
<212>  DNA
<213>  Homo sapiens

<400>  13
agggctttag actgaataac cttggggata aactgaatct ttcatattta ggaatagtag        60

ggattagtag caaaaccctc aaatacattt tagaaacaca atttcaggaa tttgggtatg       120

ttaaattcat ctagttacaa tcttttctga atctgaacag cttctcttta ttctgtagat 180

ggtggctgta ggaatctgtc gcacagatga ccacgtggtt agtggcaakc tggtgwcccc 240

ccttcctgtg attttaggcc atgaggcagc cggcatcgtg gagagtgttg gagaaggggt 300

gactacagtc aaaccaggta caggattcac actcagggaa cacgtgtggt tcaccatcca 360

ggatttccca gcctggataa ggaaaccaag gcaatgagag acgaaagtgc ttgcacaagg 420

tcaccgcgca gccaggactt caggagtttc ctctttccct ctctgcctgc ctgacccaag 480

catgtatgca ttcaggcact c 501

<210> 14
<211> 501
<212> DNA
<213> Homo sapiens

<400> 14
agggctttag actgaataac cttggggata aactgaatct ttcatattta ggaatagtag 60

ggattagtag caaaaccctc aaatacattt tagaaacaca atttcaggaa tttgggtatg 120

ttaaattcat ctagttacaa tcttttctga atctgaacag cttctcttta ttctgtagat 180

ggtggctgta ggaatctgtc acacagatga ccacgtggtt agtggcaakc tggtgwcccc 240

ccttcctgtg attttaggcc atgaggcagc cggcatcgtg gagagtgttg gagaaggggt 300

gactacagtc aaaccaggta caggattcac actcagggaa cacgtgtggt tcaccatcca 360

ggatttccca gcctggataa ggaaaccaag gcaatgagag acgaaagtgc ttgcacaagg 420

tcaccgcgca gccaggactt caggagtttc ctctttccct ctctgcctgc ctgacccaag 480

catgtatgca ttcaggcact c 501

<210> 15
<211> 18
<212> DNA
<213> Artificial Sequence

<220>
<223> probe

<220>
<221> misc_feature
<222> (11)..(11)
<223> n is a, c, g, or t

<400> 15
tctgtcgcac ngatgacc 18

<210> 16
<211> 18
<212> DNA
<213> Artificial Sequence

<220>

<223>  probe

<400>  16
tctgtcgcac ggatgacc                                                    18


<210>  17
<211>  18
<212>  DNA
<213>  Artificial Sequence

<220>
<223>  probe

<400>  17
tctgtcacac agatgacc                                                    18


<210>  18
<211>  18
<212>  DNA
<213>  Artificial Sequence

<220>
<223>  probe

<400>  18
tgtcacacag atgaccac                                                    18


<210>  19
<211>  46
<212>  DNA
<213>  Artificial Sequence

<220>
<223>  primer

<400>  19
gaaacacaat ttcaggaatt tgggtatgtt aaattcatct agttac                     46


<210>  20
<211>  25
<212>  DNA
<213>  Artificial Sequence

<220>
<223>  primer

<400>  20
ggwcaccagk ttgccactaa ccacg                                            25


<210>  21
<211>  50
<212>  DNA
<213>  Artificial Sequence

<220>
<223>  oligonucleotide

<400>  21
ggtggctgta ggaatctgtc gcacagatga ccacgtggtt agtggcaacc                 50

<210>  22
<211>  50
<212>  DNA
<213>  Artificial Sequence

<220>
<223>  oligonucleotide

<400>  22
ggtggctgta ggaatctgtc acacagatga ccacgtggtt agtggcaacc                50


<210>  23
<211>  50
<212>  DNA
<213>  Artificial Sequence

<220>
<223>  oligonucleotide

<400>  23
ggttgccact aaccacgtgg tcatctgtgc gacagattcc tacagccacc                50


<210>  24
<211>  50
<212>  DNA
<213>  Artificial Sequence

<220>
<223>  oligonucleotide

<400>  24
ggttgccact aaccacgtgg tcatctgtgt gacagattcc tacagccacc                50